# EUROPEAN PATENT APPLICATION

(11) **EP 4 685 127 A1**
(43) Date of publication of application: **28.01.2026**
(21) Application number: 24807108.6
(22) Date of filing: 09.05.2024
(51) Int. Cl.: C07C 17/363, C07C 25/13, C07C 37/055, C07C 39/27, C07C 43/225, H01L 21/3065

(54) **METHOD FOR PRODUCING AROMATIC COMPOUND HAVING FLUOROALKYL GROUP**

(30) Priority: 16.05.2023 JP 2023080848
(71) Applicant: Daikin Industries, Ltd., Osaka-shi, Osaka 530-0001 (JP)
(72) Inventor: HASHIZUME, Miyako, Osaka-Shi, Osaka 530-0001 (JP); ETO, Yusuke, Osaka-Shi, Osaka 530-0001 (JP)
(74) Representative: Hoffmann Eitle
(86) International application number: PCT/JP2024/017206
(87) International publication number: WO 2024/237156

(57) **Abstract**

Provided is a novel method that can efficiently produce an aromatic compound having a fluoroalkyl group. The method for producing a compound represented by formula (1): 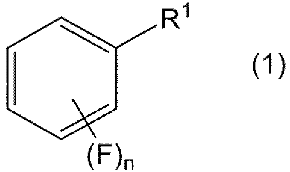 wherein each R¹ is the same or different and represents a fluoroalkyl group, and n represents an integer of 2 to 5, the method comprising a step of heating a compound represented by formula (2): 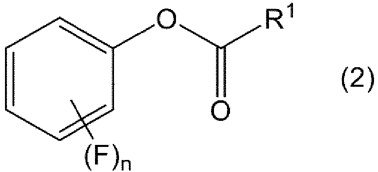 wherein R¹ and n are the same as above, the heating step satisfying either of the following: (1) using a batch process and heating to 170°C or higher, or (2) using a continuous flow process and heating to 500°C or higher.

## Description

### Technical Field

The present disclosure relates to a method for producing an aromatic compound having a fluoroalkyl group.

### Background Art

A known method for producing an aromatic compound having a fluoroalkyl group is, for example, to use phenyl trifluoroacetate or 4-fluorophenyl trifluoroacetate as a starting material, flow the starting material through a quartz glass tube while heating to 650°C, and thermally deoxidize it to obtain trifluoromethylbenzene or 1-fluoro-4-fluorophenylbenzene (see, for example, NPL 1). As a result, when phenyl trifluoroacetate is used as the starting material, the yield of the target product is 15.5%, and when 4-fluorophenyl trifluoroacetate is used as the starting material, the yield of the target product is 2.0%.

### Citation List

### Non-patent Literature

NPL 1: Journal of Fluorine Chemistry, 32 (1986) 467-470

### Summary of Invention

### Technical Problem

An object of the present disclosure is to provide a novel method that can efficiently produce an aromatic compound having a fluoroalkyl group.

### Solution to Problem

The present disclosure includes the following subject matter.

Item 1. A method for producing a compound represented by formula (1): 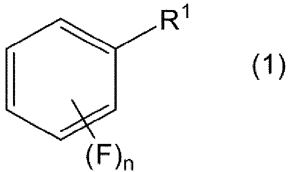 wherein each R¹ is the same or different and represents a fluoroalkyl group, and n represents an integer of 2 to 5,
the method comprising a step of heating a compound represented by formula (2): 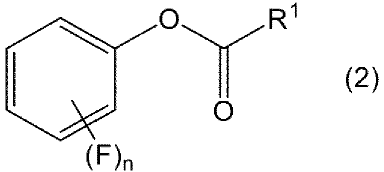 wherein R¹ and n are the same as above,
the heating step satisfying either of the following:
   (1) using a batch process and heating to 170°C or higher, or
   (2) using a gas-phase continuous flow process and heating to 500°C or higher.

Item 2. The production method according to Item 1, wherein R¹ is a perfluoroalkyl group.

Item 3. The production method according to Item 1 or 2, wherein n is 5.

Item 4. The production method according to any one of Items 1 to 3, wherein the method satisfies (1), and the heating step is performed at a gauge pressure of 0.05 to 1.00 MPa.

Item 5. The production method according to any one of Items 1 to 4, wherein the method satisfies (2), and in the heating step, a thermally conductive container is used as a reaction container.

Item 6. A composition comprising:
a compound represented by formula (1): 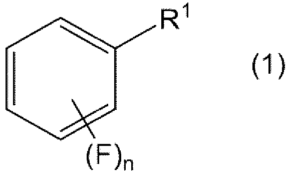 wherein each R¹ is the same or different and represents a fluoroalkyl group, and n represents an integer of 2 to 5; and
a compound represented by formula (3): 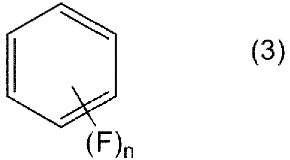 wherein n is the same as above; and/or a compound represented by formula (4): 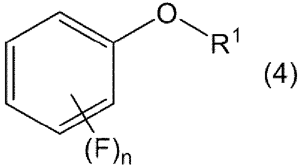 wherein R¹ and n are the same as above.

Item 7. The composition according to Item 6, comprising a total of 30 mol or less of the compound represented by formula (3) and/or the compound represented by formula (4) per mol of the compound represented by formula (1).

Item 8. The composition according to Item 6 or 7, further comprising a compound represented by formula (5): 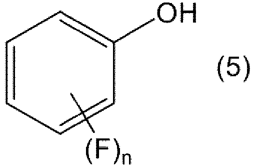 wherein n is the same as above.

Item 9. The composition according to any one of Items 6 to 8, which is for use as an etching gas, a cleaning gas, or a deposit gas.

### Advantageous Effects of Invention

According to the present disclosure, it is possible to provide a novel method for producing an aromatic compound having a fluoroalkyl group.

### Description of Embodiments

In the present specification, the terms "comprise," "contain," and "include" encompass the concepts of "comprising," "consisting essentially of," and "consisting of."

In the present specification, a numerical range indicated by "A to B" means A or more, and B or less.

In the present disclosure, "selectivity" means the ratio (mol%) of the total molar amount of the target compound contained in a reaction product (in the case of a gas-phase continuous flow process, a gas flowing out from a reactor outlet) to the total molar amount of the compounds other than the starting compound in the reaction product (in the case of a gas-phase continuous flow process, a gas flowing out from a reactor outlet).

In the present disclosure, "conversion" means the ratio (mol%) of the total molar amount of the compounds other than the starting compound contained in a reaction product (in the case of a gas-phase continuous flow process, a gas flowing out from a reactor outlet) to the molar amount of the starting compound supplied to a reactor.

In the present disclosure, "yield" means the ratio (mol%) of the total molar amount of the target compound contained in a reaction product (in the case of a gas-phase continuous flow process, a gas flowing out from a reactor outlet) to the molar amount of the starting compound supplied to a reactor.

### 1. Method for Producing Aromatic Compound Having Fluoroalkyl Group

The production method of the present disclosure is a method for producing a compound represented by formula (1): 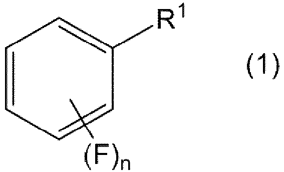 wherein each R¹ is the same or different and represents a fluoroalkyl group, and n represents an integer of 2 to 5,
the method comprising a step of heating a compound represented by formula (2): 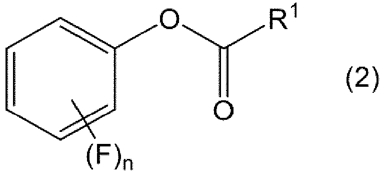 wherein R¹ and n are the same as above,
the heating step satisfying either of the following:
   (1) using a batch process and heating to 170°C or higher, or
   (2) using a gas-phase continuous flow process and heating to 500°C or higher.

### (1-1) Starting Compound (Formula (2))

In the production method of the present disclosure, the compound represented by formula (2) is a compound represented by formula (2): 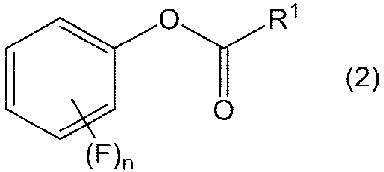 wherein each R¹ is the same or different and represents a fluoroalkyl group, and n represents an integer of 2 to 5.

In formula (2), the fluoroalkyl group represented by R¹ refers to an alkyl group in which at least one hydrogen atom is replaced by a fluorine atom.

In the present disclosure, the more fluorine atoms with high electronegativity (unlike other halogen atoms) there are, the more stable and easily produced fluoroalkyl anions are, and the more easily the bond cleavage required for the thermal deoxidation reaction occurs, making the thermal deoxidation reaction more likely to proceed. In this respect, it is preferable for the fluoroalkyl group to have a large number of fluorine atoms. Therefore, the fluoroalkyl group is preferably a perfluoroalkyl group. The perfluoroalkyl group refers to an alkyl group in which all hydrogen atoms are replaced by fluorine atoms. On the other hand, if the bond between the carbon atom in the carbonyl group and oxygen at the α-position is easily cleaved, ether and alcohol compounds as by-products are more likely to be produced. Therefore, when there is a large number of fluorine atoms, fluoroalkyl anions are stable and easily produced, and the bond cleavage required for the thermal deoxidation reaction occurs more easily, making it easier to suppress the production of these by-products.

The fluoroalkyl group may be linear, branched, or cyclic. Of these, a linear fluoroalkyl group is preferred, from the viewpoint of conversion, yield, selectivity, and the like.

The number of carbon atoms in the fluoroalkyl group is not limited, but is preferably 1 to 5**,** more preferably 1 to 4, and even more preferably 1 to 3, from the viewpoint of conversion, yield, selectivity, and the like.

Specific examples of such fluoroalkyl groups include a fluoromethyl group, a difluoromethyl group, a trifluoromethyl group, a fluoroethyl group (e.g., a 1-fluoroethyl group or a 2-fluoroethyl group), a difluoroethyl group (e.g., a 1,1-difluoroethyl group or a 2,2-difluoroethyl group), a trifluoroethyl group (e.g., a 2,2,2-trifluoroethyl group), a tetrafluoroethyl group (e.g., a 1,1,2,2-tetrafluoroethyl group or a 1,2,2,2-tetrafluoroethyl group), and a pentafluoroethyl group.

In formula (2), n refers to the number of fluorine atoms bonded to the benzene ring and represents an integer of 2 to 5. As shown in NPL 1, the presence of fluorine atoms bonded to the benzene ring significantly reduces the yield of the target product. Since this tendency is more pronounced as the number of fluorine atoms increases, it is generally assumed from NPL 1 that the reaction hardly proceeds at all when there are two or more fluorine atoms bonded to the benzene ring. In contrast, in the present disclosure, even if there are two or more fluorine atoms bonded to the benzene ring, the target product can be produced by using conditions specific to each of a batch process (requirement (1)) and a gas-phase continuous flow process (requirement (2)). That is, the present disclosure is useful in that the target product can be produced even with a large number of fluorine atoms bonded to the benzene ring. n is preferably an integer of 3 to 5, more preferably 4 or 5, and even more preferably 5.

Specific examples of the compound represented by formula (2) as the starting material that satisfies the above conditions include the following:

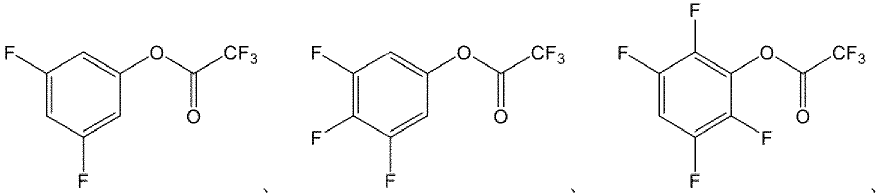

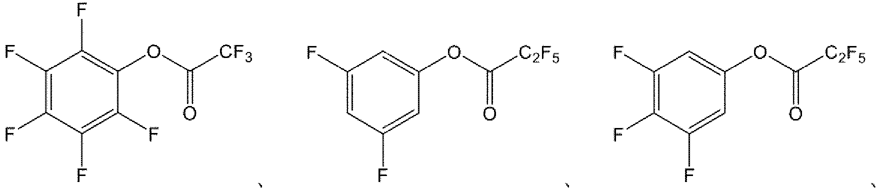

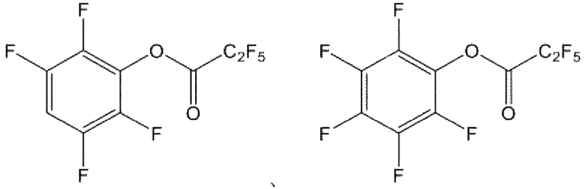

These compounds represented by formula (2) may be known or commercially available products. These compounds represented by formula (2) may be used singly or in a combination of two or more.

### (1-2) Reaction

In the reaction of the present disclosure, the -OCOR¹ group in the compound represented by formula (2) is thermally deoxidized, and the -OCO- group is eliminated by a decarboxylation reaction to form the -R¹ group.

In this case, R¹ and the hydrogen and fluorine atoms on the benzene ring remain unchanged even after the reaction of the present disclosure. That is, the structure is maintained except that the -OCOR¹ group is thermally deoxidized, and the -OCO- group is eliminated to form the -R¹ group.

As a result, the compound represented by formula (1) is obtained.

The reaction of the present disclosure can be carried out in either a batch process in which the starting material is placed all at once in the reaction container, or a gas-phase continuous flow process in which the starting material is continuously fed into the reaction container while the product is withdrawn from the reaction container. However, since the conditions vary depending on which process is used, details will be given below. Using the batch process tends to reduce the production of by-products that are difficult to separate.

The above reaction can be carried out in either liquid or gas phase.

### (1-3) Batch Process (Requirement (1))

When using a batch-type reaction in the production method of the present disclosure, the reaction container to be used is not limited as long as it can withstand the reaction, and the shape and structure are not limited. From the viewpoint of allowing the reaction to proceed even at low temperatures and facilitating the production of the compound represented by formula (1) as the target product, it is preferable to use a pressure-resistant reaction container.

In the production method of the present disclosure, the reaction container is not limited. For example, it is preferable to place the compound represented by formula (2) as a starting material in a pressure vessel, such as an autoclave, heat the vessel to 170°C or higher using a heater or the like, and allow it to react for a certain period of time. In this case, a gas-phase reaction can be used, or a liquid-phase reaction can be used. When using a liquid-phase reaction, the reaction is preferably carried out in a solvent with stirring. Since NPL 1 states that the starting material is recovered at a reaction temperature of 500°C or lower, it is an unexpected result that the decarboxylation reaction proceeds at such an extremely low temperature.

Examples of the material of the reaction container include glass, stainless steel, iron, nickel, and iron-nickel alloys.

In the production method of the present disclosure, when using a batch-type liquid-phase reaction, the solvent that can be used is not limited, and both non-polar and polar solvents can be used; however, from the viewpoint of conversion, yield, selectivity, and the like, non-polar solvents are preferred. The solvent is preferably one that does not thermally decompose at the reaction temperature. Since the reaction temperature is 170°C or higher, it is preferable to use a solvent that does not thermally decompose or has a thermal decomposition temperature of 175°C or higher. Specifically, examples of usable solvents include aliphatic organic solvents, such as hexane, cyclohexane, and heptane; aromatic organic solvents, such as benzene, toluene, xylene, mesitylene, trifluorotoluene, and chlorobenzene; aliphatic halogenated hydrocarbons, such as dichloromethane, dichloroethane, and chloroform; lactam compounds, such as N-methylpyrrolidone; ether compounds, such as tetrahydrofuran and diethyl ether; and ketone compounds, such as acetone. These solvents may be used singly or in a combination of two or more.

The amount of the non-polar solvent used is not limited as long as it is a solvent amount, and it may be an excess amount. From the viewpoint of conversion, yield, selectivity, and the like, the amount is preferably 80 to 10000 parts by mass, and more preferably 100 to 1000 parts by mass, based on 100 parts by mass of the compound represented by formula (2).

In the production method of the present disclosure, the reaction is preferably carried out in a batch-type reaction container (e.g., an autoclave) by sealing the reaction system. The reaction is preferably carried out in an inert gas atmosphere, such as nitrogen, argon, or helium.

When using the batch process in the production method of the present disclosure, the reaction temperature is 170°C or higher, preferably 180 to 300°C, and more preferably 190 to 240°C, from the viewpoint of easily suppressing the production of high-boiling-point compounds as by-products and making the thermal deoxidation reaction proceed efficiently. When the reaction temperature is less than 170°C, only side reactions proceed, and the thermal deoxidation reaction does not proceed; thus, the target product cannot be produced, resulting in poor yield and selectivity.

In the production method of the present disclosure, the reaction temperature can be raised to the highest temperature reached in one heating operation, or can be raised to the highest temperature reached stepwise in several heating operations.

In the production method of the present disclosure, the reaction pressure of the batch process refers to the pressure inside the reaction container used. In the production method of the present disclosure, although it is not limited, pressure is preferably applied from the viewpoint of easily improving the conversion, yield, selectivity, and the like even if the reaction temperature is low. Specifically, the reaction pressure is preferably 0.05 to 1.00 MPa, and more preferably 0.1 to 0.5 MPa. In the present specification, the reaction pressure generally refers to gauge pressure. When applying pressure, the reaction container is sealed, and an inert gas, such as nitrogen, argon, or helium, is introduced into the reaction system, thereby increasing the pressure inside the reaction system. By sealing the reaction container and applying pressure in this way, the fluoroalkyl group released from the starting material and the aromatic compound resulting from the release of the -OCOR¹ group from formula (2) collide to easily produce the compound represented by formula (1) as the target product.

The reaction time is not limited, and the time can be set to allow the reaction to proceed sufficiently. Specifically, the reaction can be allowed to proceed until there is no change in the composition in the reaction system.

In the production method of the present disclosure, when using a liquid-phase reaction, it is also possible to carry out the reaction in a continuous and pressurized reaction form, while extracting the liquid or extracting the gasified product, for example, by connecting a back pressure valve to a continuous stirred-tank reactor (CSTR).

After completion of the reaction, purification is optionally performed according to a common method to obtain the compound represented by formula (1).

### (1-4) Gas-Phase Continuous Flow Process (Requirement (2))

In the present disclosure, when using a gas-phase continuous flow-type reaction in which the starting material is continuously added to a reactor, and the target compound is continuously extracted from the reactor, the reaction is heated to 500°C or higher.

When using the gas-phase continuous flow process, reaction containers (reaction tubes) made of quartz or other materials that can suppress side reactions are often used, as described in NPL 1. However, the production method of the present disclosure uses a highly stable starting material of formula (2) wherein n is 2 or more. By facilitating the transfer of thermal energy, the fluoroalkyl group released from the starting material and the aromatic compound resulting from the release of the - OCOR1 group from formula (2) collide to easily produce the compound represented by formula (1) as the target product. Accordingly, although there are concerns about side reactions, it is preferable to use a thermally conductive container as the reaction container (reaction tube).

The materials that can be used for the reaction container are not limited; however, taking into consideration the reduced likelihood of side reactions, Hastelloy (nickel-based alloy), iron, stainless steel, copper, nickel, etc. are preferred.

The shape and structure of the reaction container (reaction tube) are not limited. Examples of the reaction container (reaction tube) include vertical reactors, horizontal reactors, and multitubular reactors.

In the production method of the present disclosure, when using the gas-phase continuous flow process, the reaction is preferably carried out in an inert gas atmosphere, such as nitrogen, argon, or helium.

In the production method of the present disclosure, when using the gas-phase continuous flow process, the reaction temperature is 500°C or higher, preferably 550 to 750°C, and more preferably 600 to 700°C. When the reaction temperature is lower than 500°C, almost no reaction occurs, only side reactions proceed, and the thermal deoxidation reaction does not proceed; thus, the target product cannot be produced.

In the production method of the present disclosure, when using the gas-phase continuous flow process, the reaction pressure is not limited, and the reaction can be carried out under reduced pressure, ordinary pressure, or increased pressure.

In the production method of the present disclosure, when using the gas-phase continuous flow process, the reaction time is not limited; however, from the viewpoint of conversion, yield, selectivity, and the like, the time to flow the starting material is preferably 1 to 10 minutes, more preferably 1 to 5 minutes, and even more preferably 1 to 2 minutes.

In the production method of the present disclosure, when using the gas-phase continuous flow process, the flow rate at which the starting compound is placed is not limited; however, from the viewpoint of conversion, yield, selectivity, and the like, the flow rate is preferably 1 to 100 mL/min, more preferably 2 to 50 mL/min, and even more preferably 5 to 20 mL/min.

The production method of the present disclosure can be performed in the presence of a catalyst, such as CaCl₂; however, since the reaction efficiency is not significantly affected, it is preferable to carry out the reaction in the absence of a catalyst, taking into consideration concerns about side reactions.

After completion of the reaction, purification is optionally performed according to a common method to obtain the compound represented by formula (1).

### (1-5) Target Compound (Formula (1))

The thus-obtained target compound is a compound represented by formula (1): 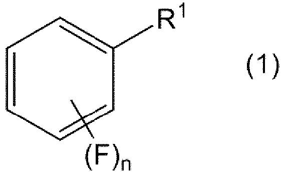 wherein each R¹ is the same or different and represents a fluoroalkyl group, and n represents an integer of 2 to 5.

In formula (1), R¹ and n are as described above.

That is, specific examples of the compound represented by formula (1), which is the target compound produced in the present disclosure, include the following:

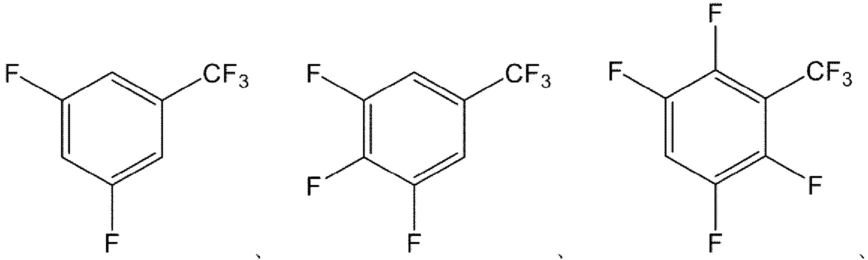

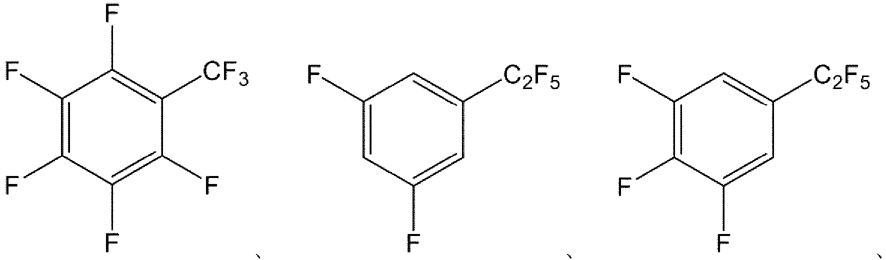

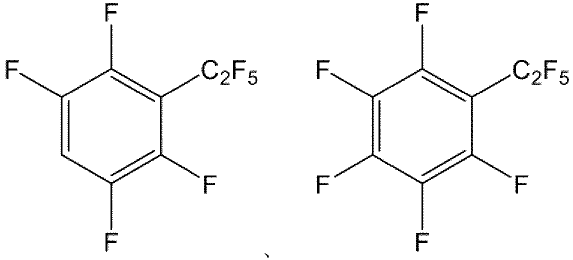

### 2. Composition

The compound represented by formula (1) can be obtained as described above, and the compound represented by formula (1) is often obtained in the form of a composition containing a compound represented by formula (1):

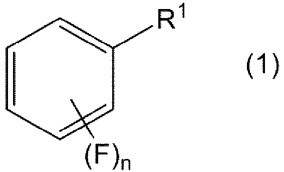

wherein each R¹ is the same or different and represents a fluoroalkyl group, and n represents an integer of 2 to 5, and a compound represented by formula (3): 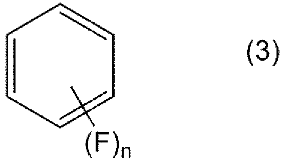 wherein n is the same as above, and/or a compound represented by formula (4): 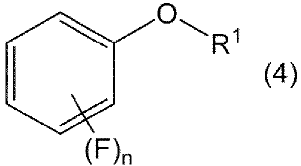 wherein R¹ and n are the same as above.

The compound represented by formula (1) is as described above.

In formula (3), n can be those mentioned above.

Therefore, examples of the compound represented by formula (3) include the following:

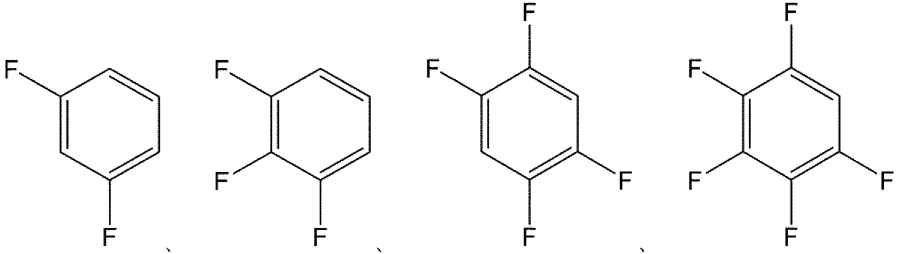

In formula (4), R¹ and n can be those mentioned above. The same applies to preferred specific examples.

Therefore, examples of the compound represented by formula (4) include the following:

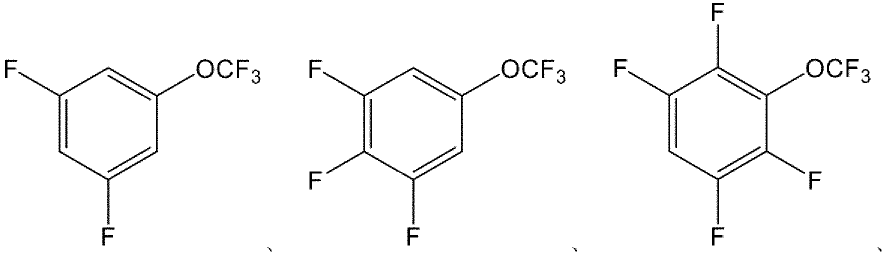

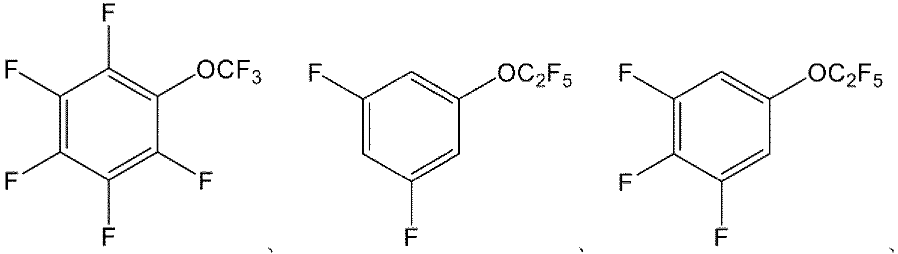

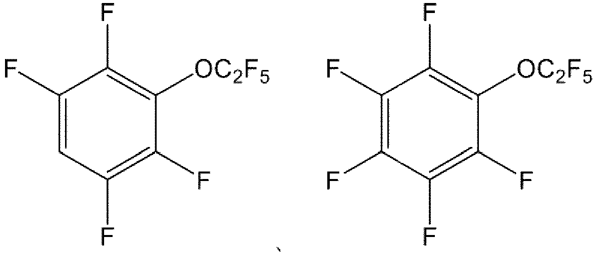

In the production method of the present disclosure, the total content of the compound represented by formula (3) and/or the compound represented by formula (4) is not limited, but can be 30 mol or less, and particularly 0.001 to 20 mol, per mol of the compound represented by formula (1).

The composition of the present disclosure can be obtained by the production method of the present disclosure. When using the batch process in the production method of the present disclosure, a composition containing the compound represented by formula (1) and the compound represented by formula (3) is likely to be obtained as the composition of the present disclosure. In this case, the content of the compound represented by formula (3) is not limited, but can be 0.10 mol or less, and particularly 0.001 to 0.10 mol, per mol of the compound represented by formula (1).

When using the gas-phase continuous flow process in the production method of the present disclosure, a composition containing the compound represented by formula (1), the compound represented by formula (3), and the compound represented by formula (4) is likely to be obtained as the composition of the present disclosure. In this case, the content of the compound represented by formula (3) is not limited, but can be 1.0 to 3.0 mol, and particularly 1.5 to 2.0 mol, per mol of the compound represented by formula (1). The content of the compound represented by formula (4) is also not limited, but can be 10.0 to 27.0 mol, and particularly 15.0 to 18.0 mol, per mol of the compound represented by formula (1).

The composition of the present disclosure may further contain a compound represented by formula (5): 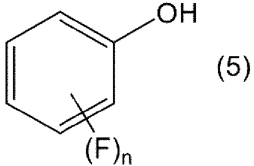 wherein n is the same as above.

In formula (5), n can be those mentioned above.

Therefore, examples of the compound represented by formula (5) include the following:

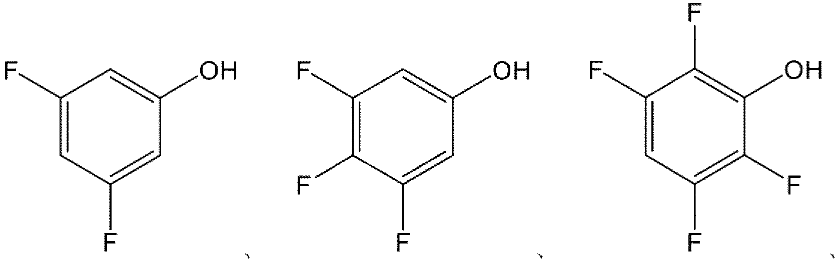

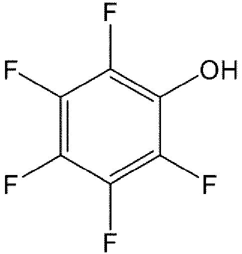

When the composition of the present disclosure contains the compound represented by formula (5), the content of the compound represented by formula (5) is not limited, but can be 5 to 80 mol, and particularly 7 to 50 mol, per mol of the compound represented by formula (1).

Since the compound represented by formula (3), the compound represented by formula (4), and the compound represented by formula (5) have retention times in gas chromatography similar to that of the compound represented by formula (1), it difficult to completely remove them even after purification.

Therefore, the composition of the present disclosure includes both purified and unpurified compositions.

When using the batch process as the production method of the present disclosure, in a purified composition, the content of the compound represented by formula (1) can be 90.00 to 99.99 volume% (particularly 95.00 to 99.98 volume%), the content of the compound represented by formula (3) can be 0.01 to 10.00 volume% (particularly 0.02 to 5.00 volume%), and the content of the compound represented by formula (5) can be 0 to 5.00 volume% (particularly 0.01 to 3.00 volume%), based on the total amount of the composition of the present disclosure, which is taken as 100 volume%; and in an unpurified composition, the content of the compound represented by formula (1) can be 1.50 to 10.00 mol% (particularly 2.00 to 8.00 mol%), the content of the compound represented by formula (3) can be 0.01 to 0.30 mol% (particularly 0.02 to 0.20 mol%), and the content of the compound represented by formula (5) can be 45.00 to 98.00 mol% (particularly 50.00 to 95.00 mol%), based on the total amount of the composition of the present disclosure, which is taken as 100 mol%.

When using the gas-phase continuous flow process as the production method of the present disclosure, in a purified composition, the content of the compound represented by formula (1) can be 85.00 to 99.98 volume% (particularly 90.00 to 99.95 volume%), the content of the compound represented by formula (3) can be 0.01 to 10.00 volume% (particularly 0.02 to 5.00 volume%), the content of the compound represented by formula (4) can be 0.01 to 10.00 volume% (particularly 0.02 to 5.00 volume%), and the content of the compound represented by formula (5) can be 0 to 5.00 volume% (particularly 0.01 to 3.00 volume%), based on the total amount of the composition of the present disclosure, which is taken as 100 volume%; and in an unpurified composition, the content of the compound represented by formula (1) can be 1.0 to 3.0 mol% (particularly 1.5 to 2.0 mol%), the content of the compound represented by formula (3) can be 2.0 to 5.0 mol% (particularly 2.5 to 4.0 mol%), the content of the compound represented by formula (4) can be 25.0 to 30.0 mol% (particularly 26.0 to 29.0 mol%), and the content of the compound represented by formula (5) can be 45.0 to 65.0 mol% (particularly 50.0 to 60.0 mol%), based on the total amount of the composition of the present disclosure, which is taken as 100 mol%.

The composition according to the present disclosure can be effectively used for various applications, such as etching gases, cleaning gases, and deposit gases.

The embodiments of the present disclosure described above can be altered in various ways in terms of embodiments and details without departing from the concept and the scope of the claims.

### Examples

With the Examples below, the features of the present disclosure are clearly described. The present disclosure is not limited to these Examples.

The substrate used in the Examples and Comparative Examples was pentafluorophenyl trifluoroacetate of formula (2) wherein R¹ is a trifluoromethyl group and n is 5.

### Examples 1 to 3 and Comparative Example 1 (Batch Process)

A stirrer and pentafluorophenyl trifluoroacetate in the amount shown in Table 1 were placed in a stainless steel (SUS) autoclave, which was used as a reaction container. Examples 1 and 2 and Comparative Example 1, which were gas-phase reactions, were left as they were, while Example 3 and Comparative Examples 1 and 2, which were liquid-phase reactions, had chlorobenzene added so that the solution concentration became 1 mol/L. Then, after sealing the reaction container, the temperature was raised to the temperature shown in Table 1, and heating was performed for the time shown in Table 1. However, for Example 1 and Comparative Example 1, the temperature was raised stepwise, as shown in Table 1. As a result, pressure was applied while heating, and the pressure in the reaction system was 0.13 MPa in Examples 1 and 2, 0.31 MPa in Example 3, and 0.20 MPa in Comparative Example 1.

After completion of the reaction, mass spectrometry was performed by gas chromatography (Shimadzu Corporation, trade name: GC-2014) according to gas chromatography/mass spectrometry (GC/MS) to confirm the product.

The results of mass spectrometry confirmed that octafluorotoluene was produced as the target product in Examples 1 to 3.

The results are shown in Table 1. In Table 1, "C7F8" refers to octafluorotoluene, "F5PhOH" refers to pentafluorophenol, "HB" refers to a high-boiling-point compound with an unknown structure, and "5Fbenzene" refers to pentafluorobenzene.

**Table 1**

| | | Example 1 | Example 2 | Example 3 | Comparative Example 1 |
|---|---|---|---|---|---|
| Experimental conditions | | | | | |
| | Reaction container | 30 cc AC | 30 ccAC | 30 cc AC | 30 cc AC |
| | Stirring | Chip | Chip | Chip | Chip |
| | Solvent | - | - | Chlorobenzene | Chlorobenzene |
| Amount of starting material | | | | | |
| | (g) | 0.71 | 0.60 | 0.26 | 0.27 |
| | (mol) | 0.003 | 0.002 | 0.001 | 0.001 |
| | Amount of solvent (ml) | - | - | 0.9 | 0.9 |
| Solution concentration (mol/L) | | - | - | 1.02 | 1.08 |
| Reaction conditions | | | | | |
| | Temperature (°C) | 50→200 | 200 | 200 | 160 |
| | Time (h) | 2.5 | 22 | 22 | 22 |
| Conversion (mol%) | | 6 | 11 | 24 | 30 |
| Selectivity (mol%) | | | | | |
| | C7F8 | 7.3 | 2.3 | 7.0 | 0.0 |
| | F5PhOH | 92.1 | 93.0 | 55.0 | 91.2 |
| | HB | 0.0 | 1.3 | 33.9 | 0.0 |
| | 5Fbenzene | 0.1 | 0.1 | 0.6 | 1.2 |
| | Others | 0.5 | 3.3 | 3.5 | 7.6 |

### Example 4 and Comparative Examples 2 to 6 (Gas-Phase Continuous Flow Process)

In Comparative Example 8, 0.75 g of CaCl₂ was added as a catalyst to a Hastelloy tube (outer diameter: 1.5 cm), which was used as a reaction tube. In Example 4 and Comparative Examples 2 to 5, the Hastelloy tube was used as is without using a catalyst. After drying at 25°C for 24 hours in a nitrogen atmosphere, the pressure was set to ordinary pressure, and pentafluorophenyl trifluoroacetate was circulated in the amount shown in Table 1. At that time, in Comparative Example 6, the contact time (W/F) of pentafluorophenyl trifluoroacetate and the CaCl₂ catalyst was adjusted to 5.00 g·sec/cc.

The reaction was performed by a gas-phase continuous flow process.

The reaction tube was heated at 200 to 650°C to start the reaction.

After 1.5 hours from the start of the reaction, the distillate that passed through an abatement tower was collected.

After completion of the reaction, mass spectrometry was performed by gas chromatography (Shimadzu Corporation, trade name: GC-2014) according to gas chromatography/mass spectrometry (GC/MS) to confirm the product.

The results of mass spectrometry confirmed that octafluorotoluene was produced as the target compound in Example 4.

The results are shown in Table 2. In Table 2, "C7F8" refers to octafluorotoluene, "F5PhOH" refers to pentafluorophenol, "5Fbenzene" refers to pentafluorobenzene, and "ether" refers to octafluoroanisole.

**Table 2**

| | | Example 4 | Comparative Example 2 | Comparative Example 3 | Comparative Example 4 | Comparative Example 5 | Comparative Example 6 |
|---|---|---|---|---|---|---|---|
| Experimental conditions | | | | | | | |
| | Reaction container | Hastelloy | Hastelloy | Hastelloy | Hastelloy | Hastelloy | Hastelloy |
| | Stirring | Flow | Flow | Flow | Flow | Flow | Flow |
| | Dilution | Nitrogen | Nitrogen | Nitrogen | Nitrogen | Nitrogen | Nitrogen |
| Amount of starting material | | | | | | | |
| | (g) | 1.60 | 2.23 | 2.23 | 2.23 | 1.60 | 1.60 |
| | (mol) | 0.006 | 0.008 | 0.008 | 0.008 | 0.006 | 0.006 |
| Catalyst CaCl₂ | | | | | | | |
| | W/F(g·sec/cc) | - | - | - | - | - | 5.00 |
| Reaction conditions | | | | | | | |
| | Temperature (°C) | 650 | 200 | 250 | 300 | 400 | 400 |
| | Flow time (min) | 13 | 17 | 17 | 17 | 13 | 13 |
| Conversion (mol%) | | 59 | 4 | 4 | 6 | 4 | 5 |
| Selectivity (mol%) | | | | | | | |
| | C7F8 | 1.7 | 0.0 | 0.0 | 0.0 | 0.0 | 0.0 |
| | F5PhOH | 55.4 | 100.0 | 100.0 | 96.0 | 100.0 | 100.0 |
| | 5Fbenzene | 3.1 | 0.0 | 0.0 | 0.0 | 0.0 | 0.0 |
| | ether | 27.8 | 0.0 | 0.0 | 0.0 | 0.0 | 0.0 |
| | Others | 12.0 | 0.0 | 0.0 | 4.0 | 0.0 | 0.0 |

### Examples 5 and 6 and Comparative Example 7

The mixtures obtained in Examples 3 and 4 were purified by a conventional method, and compositions with the flow ratio (volume ratio) shown in Table 3 were obtained as compositions of Examples 5 and 6. A composition purified from the mixture obtained in Example 3 is the composition of Example 5, and a composition purified from the mixture obtained in Example 4 is the composition of Example 6. In Comparative Example 7, commercially available octafluorotoluene was used as is. In Table 3, "C7F8" refers to octafluorotoluene, "F5PhOH" refers to pentafluorophenol, "5Fbenzene" refers to pentafluorobenzene, and "ether" refers to octafluoroanisole.

The obtained compositions were each etched using ICP (inductive coupled plasma) under the following etching conditions: discharge power: 1000 W, bias power: 300 W, pressure: 10 mTorr, electron density: 8 × 10¹⁰ to 2 × 10¹¹ cm⁻³, and electron temperature: 5 to 7 eV. The etching rates of a silicon oxide (SiO₂) film with a thickness of 1000 µm formed on a silicon substrate and an amorphous carbon film (ACL film) with a thickness of 5000 µm formed on a silicon substrate were measured, and the ratio of the etching rates of the SiO₂ film and the ACL film (etching rate of SiO₂ film/etching rate of ACL film) was taken as the selectivity with respect to ACL. The SiO₂ film was formed according to a conventional method, and the ACL film was formed based on a previously reported device (Producer (registered trademark) APFTM PECVD - Applied Materials). The resulting selectivity with respect to ACL (etching rate of SiO₂ film/etching rate of ACL film) is shown in Table 3.

**Table 3**

| | | Example 5 | Example 6 | Comparative Example 7 |
|---|---|---|---|---|
| Composition (volume%) | | | | |
| | C7F8 | 98.9 | 97.9 | 100 |
| | F5PhOH | 0.1 | 0.1 | - |
| | 5Fbenzene | 1.0 | 1.0 | - |
| | ether | - | 1.0 | - |
| Selectivity with respect to ACL | | 3.0 | 2.9 | 2.9 |

## Claims

1. A method for producing a compound represented by formula (1): 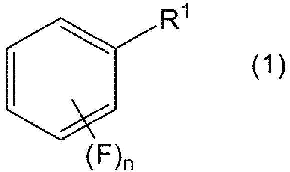 wherein each R¹ is the same or different and represents a fluoroalkyl group, and n represents an integer of 2 to 5,
the method comprising a step of heating a compound represented by formula (2): 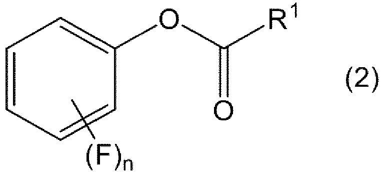 wherein R¹ and n are the same as above,
the heating step satisfying either of the following:
(1) using a batch process and heating to 170°C or higher, or
(2) using a gas-phase continuous flow process and heating to 500°C or higher.

2. The production method according to claim 1, wherein R¹ is a perfluoroalkyl group.

3. The production method according to claim 1, wherein n is 5.

4. The production method according to claim 1, wherein the method satisfies (1), and the heating step is performed at a gauge pressure of 0.05 to 1.00 MPa.

5. The production method according to claim 1, wherein the method satisfies (2), and in the heating step, a thermally conductive container is used as a reaction container.

6. A composition comprising:
a compound represented by formula (1): 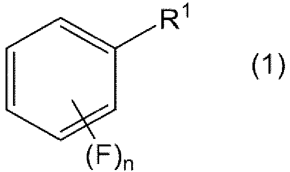 wherein each R¹ is the same or different and represents a fluoroalkyl group, and n represents an integer of 2 to 5; and
a compound represented by formula (3): 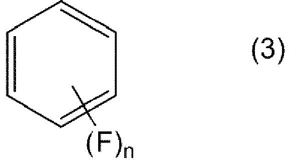
wherein n is the same as above; and/or a compound represented by formula (4): 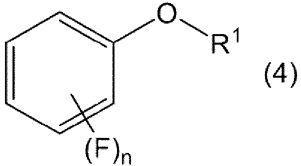
wherein R¹ and n are the same as above.

7. The composition according to claim 6, comprising a total of 30 mol or less of the compound represented by formula (3) and/or the compound represented by formula (4) per mol of the compound represented by formula (1).

8. The composition according to claim 6, further comprising a compound represented by formula (5): 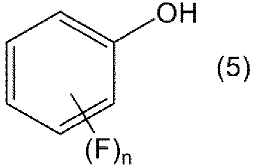 wherein n is the same as above.

9. The composition according to any one of claims 6 to 8, which is for use as an etching gas, a cleaning gas, or a deposit gas.
